# FASCICULE DE BREVET EUROPEEN

(11) **EP 1 029 535 B1**
(45) Date de publication et mention de la délivrance du brevet: **24.03.2004**
(21) Numéro de dépôt: 00400216.8
(22) Date de dépôt: 27.01.2000
(51) Int. Cl.: A61K 7/50, A61K 7/06

(54) **Compositions cosmétiques détergentes contenant un tensioactif hydroxyalkylether anionique et une silicone et leurs utilisations**
Kosmetische tensidische Zusammensetzungen, enthaltend ein anionisches Hydroxyalkylehter-Tensid und ein Silicon und deren Verwendungen
Cosmetic tensidic compositions containing anionic hydroxyalkylether surfactant and a silicone and their uses

(30) Priorité: 16.02.1999 FR 9901865
(43) Date de publication de la demande: 23.08.2000
(73) Titulaire: L'OREAL, 75008 Paris (FR)
(72) Inventeur: Garnier, Nathalie, Springfield, NJ 07081 (US); Cauwet-Martin, Danièle, 75011 Paris (FR); Restle, Serge, 95390 Saint Prix (FR)
(74) Mandataire: Le Blainvaux Bellegarde, Françoise

(56) Documents cités:
- EP-A- 0 864 638
- WO-A-94/17783
- WO-A-98/00486
- US-A- 5 490 955

## Description

La présente invention concerne de nouvelles compositions cosmétiques comprenant dans un milieu cosmétiquement acceptable au moins un tensioactif anionique de hydroxyalkyléther carboxylique et au moins une silicone.

Pour le nettoyage et/ou le lavage des cheveux et/ou de la peau, l'utilisation de compositions détergentes (shampooing ou gel-douche) à base essentiellement d'agents tensioactifs classiques de type notamment anionique, non ionique et/ou amphotère, mais plus particulièrement de type anionique, est courante. Ces compositions sont appliquées sur cheveux ou peau mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur les matières kératiniques des dommages plus ou moins marqués liés en particulier à l'élimination progressive des lipides ou protéines contenues dans ou à la surface de ces dernières.

Aussi, pour améliorer les propnétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes.

Toutefois, et malgré les progrès réalisés récemment dans le domaine des shampooings à base de polymères cationiques et/ou de silicone, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoquées ci-avant, de meilleures performances.

Les tensioactifs anioniques de type hydroxyalkyléther carboxylique ont déjà été préconisés dans des compositions cosmétiques détergentes. Ils ont été décrits par exemple dans les demandes de brevet J63280798, J08268487 et J08269482.

Les compositions de lavage des cheveux utilisant ces tensioactifs seuls ne conduisent pas à de propriétés cosmétiques satisfaisantes.

L'invention a donc pour but de proposer des compositions cosmétiques détergentes présentant des propriétés cosmétiques améliorées, en particulier le démêlage, le lissage et la douceur des cheveux.

Or, la demanderesse a maintenant trouvé que l'association de silicones particulières et d'un tensioactif anionique de hydroxyalkyléther carboxylique permettait d'atteindre ces buts.

Ces nouvelles compositions permettent de mieux déposer ces silicones sur les matières kératiniques (notamment les cheveux) qu'une composition contenant des tensioactifs anioniques classiques tels que les sels de alkyléthercarboxylates, ceci sans aspect visuel gras ou toucher gras.

Les compositions conformes à l'invention confèrent aux matières kératiniques notamment les cheveux, un remarquable effet traitant qui se manifeste notamment par une facilité de démêlage, ainsi qu'un apport de volume, de légèreté, de lissage, de douceur et de souplesse et de discipline sans aucune sensation de toucher chargé.

L'invention a ainsi pour objet une composition cosmétique détergente, caractérisée en ce qu'elle comprend, dans un milieu cosmétiquement acceptable, au moins un tensioactif anionique de type hydroxy-2 alkyléthercarboxylique ou ses sels de stucture (I) ci-après et au moins une silicone choisie parmi :
(i) les polyalkylsiloxanes, les polyarylsiloxanes, les polyalkylarylsiloxanes volatiles ou non volatiles, linéaires, ramifiés ou cycliques, réticulés ou non réticulés.
(ii) les polysiloxanes, comportant dans leur structure générale, un ou plusieurs groupements organofonctionnels choisis parmi :
   a) des groupements aminés substitués ou non
   b) des groupements (per)fluorés,
   c) des groupements thiols ;
   d) des groupements carboxylates,
   e) des groupements hydroxylés,
   f) des groupements alcoxylés
   g) des groupements acyloxyalkyls
   h) des groupements amphotères;
   i) des groupements bisulfites.
   j) des groupements hydroxyacylamino,
   k) des groupements acide carboxyliques
   l) des groupements sulfoniques
   m) des groupements sulfates ou thiosulfates;
(iii) les copolymères blocs linéaires polysiloxane(A)-polyoxyalkylène(B) de type (A-B)ₙ avec n >3 ;
(iv) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ;
(v) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ;
(vi) ou leurs mélanges.

Un autre objet de l'invention concerne un procédé de traitement des matières kératiniques, telles que les cheveux, caractérisé en ce qu'il consiste à appliquer sur lesdites matières des compositions cosmétiques selon l'invention.

L'invention a encore pour objet l'utilisation d'une composition selon l'invention pour le démêlage, le lissage des cheveux, pour apporter du volume, de la légèreté, de la douceur, de la souplesse et de la discipline aux cheveux.

L'invention a encore pour objet l'utilisation de tensioactifs de type hydroxy-2 alkyl éther carboxylique de stucture (l) ci-après dans ou pour la fabrication de compositions cosmétiques détergentes comprenant au moins une silicone définie ci-dessus.

Selon la présente invention, par matières kératiniques, on comprend les cheveux, les cils, les sourcils, la peau, les ongles, les muqueuses ou le cuir chevelu et plus particulièrement les cheveux.

Les différents objets de l'invention vont maintenant être détaillés. L'ensemble des significations et définitions des composés utilisés dans la présente invention données ci-dessous sont valables pour l'ensemble des objets de l'invention.

Les tensioactifs anioniques de type acide hydroxy-2 alkyl éther carboxylique et ses sels peuvent avoir la structure suivante : 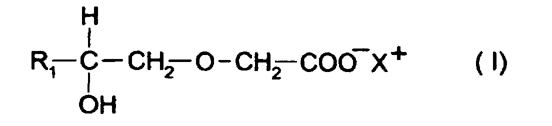 dans laquelle:
R₁ désigne plus particulièrement un radical alkyle saturé ou insaturé, linéaire ou ramifié comportant de 8 à 30 atomes de carbone.

X désigne l'hydrogène ou un cation minéral ou organique tel que :
ceux issus d'un métal alcalin (par exemple Na⁺, K⁺), NH₄⁺, les ammoniums issus des aminoacides basiques tels que la lysine, l'arginine, la sarcosine, l'ornithine, la citrulline ou bien encore des amino-alcools tels que la monoéthanolamine, la diéthanolamine, la triéthanolamine, la glucamine, la N-méthyl glucamine, l'amino-3 propanediol-1,2.

Des acides hydroxy-2 alkyl éther carboxyliques préférés selon la présente invention sont des composés de formule (I) dans laquelle R₁ désigne plus particulièrement un radical alkyle saturé ou insaturé, linéaire ou ramifié comportant de 8 à 18 atomes de carbone.

Encore plus particulièrement, R₁ désigne un radical ou un mélange de radicaux en C₈-C₁₈ notamment dérivés de coprah.

Parmi les tensioactifs de formule (I), on peut citer le produit commercialisé sous la dénomination BEAULIGHT SHAA par la société SANYO.

Selon l'invention, le tensioactif anionique de type hydroxy-2 alkyl éther carboxylique peut représenter de 1 % à 30 % en poids, de préférence de 3 % à 15 % en poids par rapport au poids total de la composition finale.

Les silicones utilisables conformément à l'invention peuvent être solubles ou insolubles dans l'eau ou dans la composition finale. Elles peuvent être volatiles ou non volatiles.

Les silicones utilisables conformément à l'invention sont en particulier insolubles dans l'eau et peuvent se présenter sous forme d'huiles, de cires, de résines ou de gommes.

Les silicones sont définis plus en détail dans l'ouvrage de Walter NOLL "Chemistry and Technology of Silicones" (1968) Academic Press.

Lorsqu'ils sont volatiles, les polyalkylsiloxanes, les polyarylsiloxanes, les polyalkylarylsiloxanes sont plus particulièrement choisies parmi ceux possédant un point d'ébullition compris entre 60°C et 260°C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE® 70045 V 2" par RHODIA CHIMIE, le décaméthylcyclopentasiloxane commercialisé sous le nom de "VOLATILE SILICONE 7158" par UNION CARBIDE, "SILBIONE® 70045 V 5" par RHODIA CHIMIE, ainsi que leurs mélanges.
   On peut également citer les cyclocopolymères du type diméthylsiloxanes/méthylalkylsiloxane, tel que la "SILICONE VOLATILE FZ 3109" commercialisée par la société UNION CARBIDE, de structure chimique : 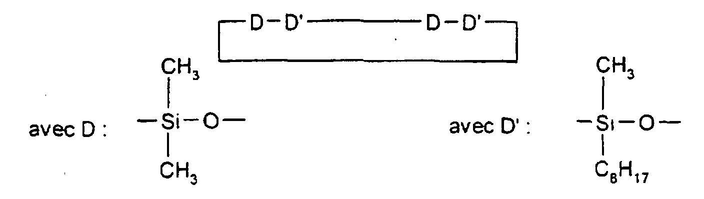
   On peut également citer les mélanges de silicones cycliques avec des composés organiques dérivés du silicium, tels que le mélange d'octaméthylcyclotétrasiloxane et de tétra(triméthylsilyl)pentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence les polyalkylsiloxanes, les polyarylsiloxanes, les polyalkylarylsiloxanes non volatiles sous forme d'huiles, de gommes de silicone ou de résines de silicone ;

Parmi les polyalkylsiloxanes, on peut citer principalement :
- les polydiméthylsiloxanes linéaires à groupements terminaux triméthylsilyle, comme par exemple, et à titre non limitatif, les huiles SILBIONE® de la série 70047 commercialisées par RHODIA CHIMIE, l'huile SILBIONE® 47 V 500 000 de RHODIA CHIMIE ou certaines VISCASIL de la GENERAL ELECTRIC ;
- les polydiméthylsiloxanes linéaires à groupements terminaux hydroxydiméthylsilyle telles que les huiles de la série 48 V de RHODIA CHIMIE.

Dans cette classe de polyalkylsiloxanes, on peut également mentionner les polyalkylsiloxanes vendus par la société GOLDSCHMIDT sous les dénominations commerciales ABILWAX® 9800 et ABILWAX® 9801 qui sont des polyalkyl(C₁-C₂₀)siloxanes.

Les polyalkylsiloxanes ont de préférence une viscosité supérieure ou égale à 500 cSt. (5 cm²/s).

La viscosité des silicones est mesurée à 25°C selon la norme ASTM 445 Appendice C.

Parmi les polyalkylarylsiloxanes, on peut citer les polydiméthylméthylphénylsiloxanes ou les polydiméthyldiphénylsiloxanes, linéaires ou ramifiés tels que le produit DC 556 COSMETIC GRADE FLUID de DOW CORNING.

Les gommes de silicone, conformes à l'invention, sont des polysiloxanes de masse moléculaire moyenne en nombre comprise entre 200.000 et 5.000.000, utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On cite, par exemple, les composés suivants :
- polydiméthylsiloxane,
- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/(méthylvinylsiloxane)],

On peut citer, par exemple, les mélanges suivants :
1) les mélanges formés à partir d'un polydiméthylsiloxane hydroxylé en bout de chaîne (DIMETHICONOL selon la nomenclature CTFA) et d'un polydiméthylsiloxane cyclique (CYCLOMETHICONE selon la nomenclature CTFA) tels que le produit Q2 1401 vendu par la société DOW CORNING ;
2) les mélanges formés à partir d'une gomme de polydiméthylsiloxane avec une silicone cyclique tels que le produit SF 1214 SILICONE FLUID de GENERAL ELECTRIC qui est une gomme SE 30 de poids moléculaire 500.000 solubilisée dans la SF 1202 SILICONE FLUID (décaméthylcyclopentasiloxane) ;
3) les mélanges de deux polydiméthylsiloxanes (PDMS) de viscosité différente, notamment d'une gomme PDMS et d'une huile PDMS tels que les produits SF 1236 et CF 1241 de la GENERAL ELECTRIC. Le produit SF 1236 est le mélange d'une huile SE 30 définie ci-dessus de viscosité 20 m²/s et d'une huile SF 96 de viscosité 5.10⁻⁵m²/s (15% de gomme SE 30 et 85% d'huile SF 96). Le produit CF 1241 est le mélange d'une gomme SE 30 (33%) et d'une PDMS (67%) de viscosité 10⁻³ m²/s.

Les résines de silicone conformes à l'invention sont de préférence des systèmes siloxaniques réticulés renfermant les unités :
R₂SiO_{2/2}, RSiO_{3/2}, SiO_{4/2}, dans lesquelles R désigne un groupe hydrocarboné possédant 1 à 6 atomes de carbone ou un groupement phényle. Parmi ces produits, ceux particulièrement préférés sont ceux où R désigne un radical alkyle inférieur (C1-C4) ou phényle.

Parmi ces résines, on peut citer le produit vendu sous le nom DOW CORNING 593 par DOW CORNING ou ceux vendus sous le nom SILICONE FLUID SS 4267 par la GENERAL ELECTRIC et qui sont des diméthyl/triméthylpolysiloxanes.

Les polysiloxanes comportant dans leur structure générale, un ou plusieurs groupements organofonctionnels sont notamment les polyalkylsiloxanes, les polyarylsiloxanes, les polyalkylarylsiloxanes tels que définis précédemment et comportant un ou plusieurs groupements organofonctionnels directement fixés sur la chaîne siloxanique ou fixés par l'intermédiaire d'un radical hydrocarboné.
Les polysiloxanes à groupements organofonctionnels de l'invention sont ceux comportant :
a) des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ou aminoalkyl(C₁-C₄)aminoalkyle(C₁-C₄). On utilise plus particulièrement les silicones dénommées amodiméthicone et triméthylsilylamodiméthicone selon la dénomination CTFA ;
b) des groupements (per)fluorés, comme les groupements trifluoroalkyls tels que, par exemple, ceux vendus par SHIN ETSU sous le nom FL 100 ;
c) des groupements thiols ;
d) des groupements carboxylates, tels que les produits décrits dans le brevet européen EP 185 507 de CHISSO CORPORATION ;
e) des groupements hydroxylés, tels que les polyorganopolysiloxanes à fonction hydroxyalkyle en C2-C12 décrits dans la demande de brevet français FR 85-16334 et en particulier les polyorganopolysiloxanes à fonction γ- hydroxypropyle ;
f) des groupements alcoxylés comportant au moins 12 atomes de carbone, tels que le produit SILICONE COPOLYMER F755 de SWS SILICONES et les produits ABILWAX® 2428, ABILWAX® 2434, ABILWAX® 2440 de la société GOLDSCHMIDT.
g) des groupements acyloxyalkyls comportant au moins 12 atomes de carbone, tels que les polyorganosiloxanes décrits dans la demande de brevet français FR 88-17433 et en particulier les polydiméthylsiloxanes à fonction stéaroyloxypropyle.
h) des groupements amphotères tels que les polydiméthylsiloxanes à groupements propylglycolbétaïne comme les produits ABIL E200, B995, BC-1600, BC-1602 de GOLDSCHMIDT ou à groupements alkyl phosphobétaïne comme les produits PECOSIL SMQ-40 et SPB-1240 de PHOENIX CHEMICAL.
i) des groupements bisulfites
j) des groupements hydroxyacylamino, comme les polydiméthylsiloxanes à groupement hydroxyacylaminopropyle décrits dans la demande EP 342 834. On peut citer par exemple le produit Q2-8413 de la société DOW CORNING.
k) des groupements acide carboxylique ou leurs sels tels que les produits commercialisés par BASF sous la dénomination DENSODRIN OF ou par la société WACKER sous la dénomination huile MS 642.
l) des groupements sulfoniques
m) des groupements sulfates ou thiosulfates comme les polydiméthylsiloxanes à groupements α,ω-thiosulfate tels que les produits ABIL S255, S32 et S201 de GOLDSCHMIDT.

Les copolymères blocs ayant un bloc linéaire polysiloxane-polyoxyalkylène de type (A-B)ₙ utilisés dans le cadre de la présente invention ont de préférence la formule générale suivante :

([Y(R₂SiO)ₐ R'₂SiYO] [(CₙH₂ₙO)_{b}])_{c} (V)

dans laquelle :
- R et R', identiques ou différents, représentent un radical hydrocarboné monovalent ne contenant pas d'insaturation aliphatique,
- n est un nombre entier allant de 2 à 4,
- a est un nombre entier supérieur ou égal à 5, de préférence compris entre 5 et 200 et encore plus particulièrement entre 5 et 100.
- b est un nombre entier supérieur ou égal à 4, de préférence compris entre 4 et 200 et encore plus particulièrement entre 5 et 100.
- c est un nombre entier supérieur ou égal à 4, de préférence compris entre 4 et 1000 et encore plus particulièrement entre 5 et 300.

- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10 % environ à 95 % environ en poids du copolymère bloc,
- le poids moléculaire moyen en poids du copolymère bloc étant d'au moins 3.000 et de préférence compris entre 5000 et 1000000 et encore plus particulièrement entre 10000 et 200000.

R et R' sont préférentiellement choisis parmi le groupe comprenant les radicaux alkyls comme par exemple les radicaux méthyle, éthyle, propyle, butyle, pentyle, hexyle, octyle, décyle, dodécyle, les radicaux aryles comme par exemple phényle, naphtyle, les radicaux aralkyles comme par exemple benzyle, phényléthyle, les radicaux tolyle, xylyle et cyclohexyle.

Y est de préférence -R"-, -R"-CO-, -R"-NHCO-, -R"-NH-CO-NH-R'"-NHCO―, - R"-OCONH-R'''-NHCO-, où R" est un groupe alkylène divalent comme par exemple l'éthylène, le propylène ou le butylène et R"' est un groupe alkylène divalent ou un groupe arylène divalent comme -C₆H₄-, -C₆H₄-C₆H₄-, -C₆H₄-CH₂-C₆H₄-, -C₆H₄-C(CH₃)₂-C₆H₄-.

Encore plus préférentiellement, Y représente un radical alkylène divalent, plus particulièrement le radical -CH₂-CH₂-CH₂- ou le radical C₄H₈.

La préparation des copolymères blocs mis en oeuvre dans le cadre de la présente invention est décrite dans la demande européenne EP 0 492 657 A1.

Les polymères à squelette organique non-siliconé greffé par des monomères contenant un polysiloxane, conformes à l'invention, sont choisis plus préférentiellement parmi ceux décrits dans les brevets US 4,693,935, US 4,728,571 et US 4,972,037 et les demandes de brevet EP-A-0 412 704, EP-A-0 412 707, EP-A-0 640 105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Des exemples de polymères à squelette polysiloxanique greffé par des monomères organiques non siliconés convenant à la mise en oeuvre de la présente invention, ainsi que leur mode particulier de préparation, sont notamment décrits dans les demandes de brevets EP-A- 0 582 152, WO 93/23009 et WO 95/03776.

Selon l'invention, toutes les silicones peuvent également être utilisées sous forme d'émulsions ou de microémulsions.

Les silicones particulièrement préférées conformément à l'invention sont :
- les polydiméthylsiloxanes à groupements terminaux triméthylsilyle telles que les huiles ayant une viscosité comprise entre 0,2 et 2,5 m²/s à 25° C, telles que les huiles de la séries DC200 de DOW CORNING en particulier celle de viscosité 60 000 cSt, des séries SILBIONE® 70047 et 47 et plus particulièrement l'huile SILBIONE® 70 047 V 500 000 commercialisées par la société RHODIA CHIMIE, ou l'huile de silicone AK 300.000 de la société WACKER,
- les polydiméthylsiloxanes à groupements terminaux diméthylsilanols tels que les diméthiconols ;
- les polysiloxanes à groupements aminés tels que les amodiméthicones ou les triméthylsilylamodiméthicones ;

Selon l'invention, la ou les silicones peuvent représenter de 0,001 % à 10 % en poids, de préférence de 0,005 % à 5 % en poids, et encore plus préférentiellement de 0,01 % à 3 % en poids, du poids total de la composition finale.

Les compositions de l'invention contiennent en outre avantageusement au moins un autre agent tensioactif qui est généralement présent en une quantité comprise entre 0,1% et 40% en poids environ, de préférence entre 3% et 30% et encore plus préférentiellement entre 5% et 20%, par rapport au poids total de la composition.

Cet agent tensioactif peut être choisi parmi les agents tensioactifs anioniques, amphotères, non-ioniques, cationiques ou leurs mélanges.

Les tensioactifs additionnels convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersulfates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères additionnels, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₅-CONHCH₂CH₂-N(B)(C) (3)

dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂- CHOH - SO3H
R₅ désigne un radical alkyle d'un acide carboxylique présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 7ème édition, 1997, sous les dénominations Disodium Cocoamphodiacetate, Disodium Lauroamphodiacetate, Disodium Capryloamphodiacetate, Disodium Caproamphodiacetate, Disodium Coco-amphodipropionate, Disodium Lauroamphodipropionate, Disodium Caproamphodipropionate, Disodium Capryloamphodipropionate, Lauroamphodipropionic acid, Coco-amphodipropionic acid.
A titre d'exemple on peut citer le disodium cocoamphodiacetate commercialisé sous la dénomination commerciale MIRANOL® C2M concentré par la société RHODIA CHIMIE.

Dans les compositions conformes à l'invention, on utilise de préférence des mélanges d'agents tensioactifs et en particulier des mélanges d'agents tensioactifs anioniques ou des mélanges d'agents tensioactifs anioniques et d'agents tensioactifs amphotères ou non ioniques.

On utilise de préférence comme agent tensioactif anionique additionnel les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélanges avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;
- soit un agent tensioactif amphotère tel que les alkylbétaïnes en particulier la cocobétaïne commercialisée sous la dénomination "DEHYTON® AB 30" en solution aqueuse à 32 % de MA par la société HENKEL ou tel que les alkyl (C₈-C₂₀) amidoalkyl (C₁-C₆) bétaïnes en particulier la TEGOBETAINE® F 50 commercialisée par la société GOLDSCHMIDT.

On peut également utiliser des tensioactifs cationiques parmi lesquels on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique.

Le(s) agent(s) tensioactif(s) anionique(s) différents des acides hydroxy-2alkyléther carboxyliques sont généralement présents à raison de 1 à 30 % en poids, de préférence de 3 à 15 % en poids, par rapport au poids total de la composition.

Le(s) agent(s) tensioactif(s) amphotère(s) ou non ioniques sont généralement présents à raison de 0,5 à environ 15% en poids, de préférence de 1 à 5% en poids, par rapport au poids total de la composition.

La quantité et la qualité des tensioactifs sont celles suffisantes pour conférer à la composition finale un pouvoir moussant et/ou détergent satisfaisant.

Dans la composition selon la présente invention, la totalité des tensioactifs détergents représente généralement de 4 à 50% en poids et de préférence de 6 à 35% en poids et plus particulièrement de 8 à 25% en poids par rapport au poids total de la composition.

La composition de l'invention peut également contenir au moins un additif choisi parmi les épaississants, les parfums, les agents nacrants, les conservateurs, les filtres solaires, les polymères anioniques ou non ioniques ou amphotères, les polymères cationiques, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les huiles végétales, les huiles minérales et les huiles de synthèse, les agents antipelliculaires et tout autre additif classiquement utilisé dans le domaine cosmétique qui n'affecte pas la stabilité et les propriétés des compositions selon l'invention.

Ces additifs sont présents dans la composition selon l'invention dans des proportions pouvant aller de 0 à 50% en poids par rapport au poids total de la composition. La quantité précise de chaque additif est déterminée facilement par l'homme du métier selon sa nature et sa fonction.

Selon un mode préféré de l'invention, les compositions selon l'invention comprennent en outre un ou plusieurs polymères cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL.
On peut également utiliser les polymères qui sont constitués de motifs récurrents répondant à la formule : 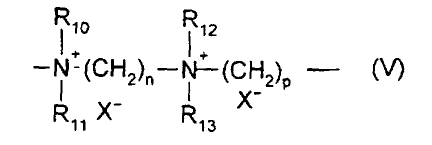 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Selon l'invention, le ou les polymères cationiques peuvent représenter de 0,001 % à 10% en poids, de préférence de 0,005% à 5% en poids, et encore plus préférentiellement de 0,01 % à 3 % en poids, du poids total de la composition finale.

Le milieu cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.

De préférence , la composition comprend de 50 à 95 % en poids d'eau par rapport au poids total de la composition.

Les compositions détergentes selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions selon l'invention peuvent contenir également des synergistes de mousses tels que des 1,2-alcanediols en C₁₀-C₁₈ ou des alcanolamides gras dérivés de mono ou de diéthanolamine.

Les compositions conformes à l'invention peuvent être utilisées pour le lavage et le traitement des matières kératiniques telles que les cheveux, la peau, les cils, les sourcils, les ongles, les lèvres, le cuir chevelu et plus particulièrement les cheveux.

En particulier, les compositions détergentes selon l'invention sont des shampooings, des gels-douche et des bains moussants.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de produits démaquillants.

Les compositions selon l'invention peuvent se présenter sous forme de gel, de lait, de crème, d'émulsion, de lotion épaissie ou de mousse et être utilisées pour la peau, le cuir chevelu, les ongles, les cils, les lèvres et plus particulièrement les cheveux.

Ces compositions détergentes sont de préférence moussantes et le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombent dans une éprouvette ayant un diamètre de 50 mm et contenant 50 ml de la composition à tester. La mesure est faite 5 minutes après l'arrêt de l'écoulement de la composition.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage en particulier avec de l'eau.

Ainsi, ce procédé selon l'invention permet le traitement, le soin, le lavage ou le démaquillage de la peau, des cheveux ou de toute autre matière kératinique.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits. Dans les exemples, MA signifie matière active.

### EXEMPLE 1 :

On a réalisé deux compositions de shampooings, l'une conforme à l'invention (composition A) et l'autre comparative (composition B) :

| | A (invention) | B |
|---|---|---|
| - 2-(2-hydroxylauryloxy)acétate de sodium an solution aqueuse à 30% de matière active (BEAULIGHT SHAA de SANYO) | 10 g MA | - |
| - Alkyl (C₁₂-C₁₄)éther sulfate de sodium oxyéthyléné à 2,2 moles d'oxyde d'éthylène en solution aqueuse à 70% de MA | 5 gMA | 5 gMA |
| - Lauryl éther carboxylate de sodium à 4,5 OE en solution aqueuse à 22% de matière active (AKYPOSOFT 45 NV de KAO) | - | 10 gMA |
| - Polydiméthylsiloxane en émulsion non ionique aqueuse à 50% de MA (DC2-1691 de DOW CORNING) | 2,5 g MA | 2,5 g MA |
| - Ether de myristylglycol et d'alcool cétyl- stéarylique oxyéthyléné à 60 moles d'oxyde d'éthylène (ELFACOS GT 282S de AKZO) | 1 g | 1 g |
| - Conservateurs | qs | qs |
| pH | 6,6 | 6,6 |
| -Eau déminéralisée qsp | 100 g | 100 g |

On effectue un shampooing en appliquant environ 1 g de la composition A sur des mèches de 2,5 g de cheveux décolorés (SA20) préalablement mouillés. On fait mousser le shampooing, on laisse pauser pendant 10 minutes puis on rince abondamment à l'eau.
On sèche les mèches pendant 10 minutes à 60°C.
On procède selon le même mode opératoire que ci-dessus avec la composition comparative B.

Un panel d'experts a évalué l'aspect des cheveux séchés.
Le test utilisé a pour objet le classement, par un jury, de chaque série de 2 échantillons en attribuant la note 1 pour la mèche qui se démêle le mieux qui est la plus douce et la plus lisse et 2 pour l'autre. Les 2 mèches de la même série sont présentées simultanément au juge. L'analyse statistique des résultats est effectuée à l'aide des tables de A. KRAMER (Food Technology 17 - (12), 124 - 125 1963).
Tous les experts indiquent que les cheveux traités avec la composition A selon l'invention sont significativement plus doux, plus lisses et se démêlent facilement que ceux traités avec la composition B.

### EXEMPLE 2 :

On a réalisé deux compositions de shampooings, l'une conforme à l'invention (composition A) et l'autre comparative (composition B) :

| | A | B |
|---|---|---|
| -2-(2-hydroxylauryloxy)acétate de sodium en solution aqueuse à 30% MA (BEAULIGHT SHAA de SANYO) | 10 g MA | - |
| - Alkyl (C₁₂-C₁₄)éther sulfate de sodium oxyéthyléné à 2,2 moles d'oxyde d'éthylène en solution aqueuse à 70% de MA | 4 gMA | 14 gMA |
| - Hexadimethrine chloride en solution aqueuse à 60% MA (MEXOMER PO de CHIMEX) | 0,6 g MA | 0,6 g MA |
| - Polydiméthylsiloxane en émulsion non ionique aqueuse à 50% de MA (DC2-1691 de DOW CORNING) | 2,5 g MA | 2,5 g MA |
| - Gomme de xanthane (KELTROL T de NUTRASWEET KELCO) | 1 g | 1 g |
| - Conservateurs | qs | qs |
| -Eau déminéralisée qsp | 100 g | 100 g |

On effectue un shampooing en appliquant environ 1 g de la composition A sur des mèches de 2,5 g de cheveux décolorés (SA20) préalablement mouillés. On fait mousser le shampooing, on laisse pauser pendant 10 minutes puis on rince abondamment à l'eau.
On sèche les mèches pendant 10 minutes à 60°C.
On procède selon le même mode opératoire que ci-dessus avec la composition comparative B.

Un panel d'experts a évalué l'aspect des cheveux séchés.
Tous les experts indiquent que les cheveux traités avec la composition A selon l'invention sont significativement plus doux et se démêlent facilement que ceux traités avec la composition B.

### EXEMPLE 3 :

On a réalisé une composition de shampooing conforme à l'invention :

| | |
|---|---|
| | |
| - 2-(2-hydroxylauryloxy)acétate de sodium en solution aqueuse à 30% de matière active (MA) (BEAULIGHT SHAA de SANYO) | 5 g MA |
| - Alkyl (C₁₂-C₁₄)éther sulfate de sodium oxyéthyléné à 2,2 moles d'oxyde d'éthylène en solution aqueuse à 70% de MA | 15 gMA |
| - Cocoylbétaïne en solution aqeuse à 30% MA | 5 gMA |
| - Polydiméthylsiloxane de viscosité 300 000 cSt | 1,5 g |
| - Gomme de xanthane (KELTROL T de NUTRASWEET KELCO) | 1 g |
| - Conservateurs | qs |
| -Eau déminéralisée qsp | 100 g |

Les cheveux traités avec la composition selon l'invention sont doux, lisses et se démêlent facilement.

## Revendications

1. Composition cosmétique détergente, **caractérisée par le fait qu'**elle comprend, dans un milieu cosmétiquement acceptable, au moins un tensioactif anionique de type acide hydroxy-2 alkylcarboxylique ou ses sels présentant la structure suivante : 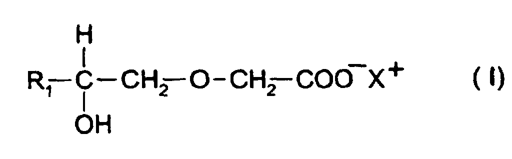
R₁ désigne un radical alkyle saturé ou insaturé, linéaire ou ramifié comportant de 8 à 30 atomes de carbone.
X désigne l'hydrogène ou un cation minéral ou organique tel que ceux issus d'un métal alcalin, NH₄⁺, les ammoniums issus des aminoacides basiques ou des amino-alcools, et au moins une silicone choisie parmi :
(i) les polyalkylsiloxanes, les polyarylsiloxanes, les polyalkylarylsiloxanes volatiles ou non volatiles, linéaires, ramifiés ou cycliques, réticulés ou non réticulés.
(ii) les polysiloxanes, comportant dans leur structure générale, un ou plusieurs groupements organofonctionnels choisis parmi :
a) des groupements aminés substitués ou non
b) des groupements (per)fluorés,
c) des groupements thiols ;
d) des groupements carboxylates,
e) des groupements hydroxylés,
f) des groupements alcoxylés
g) des groupements acyloxyalkyls
h) des groupements amphotères;
i) des groupements bisulfites.
j) des groupements hydroxyacylamino,
k) des groupements acide carboxylique
l) des groupements sulfoniques
m) des groupements sulfates ou thiosulfates;
(iii) les copolymères blocs linéaires polysiloxane(A)-polyoxyalkylène(B) de type (A-B)ₙ avec n >3 ;
(iv) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ;
(v) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ;
(vi) ou leurs mélanges.

2. Composition selon la revendication 1, **caractérisée par le fait que** X désigne Na⁺, K⁺, NH₄⁺, les ammoniums issus de la lysine, l'arginine, la sarcosine, l'ornithine, la citrulline ou issus de la monoéthanolamine, la diéthanolamine, la triéthanolamine, la glucamine, la N-méthyl glucamine ou l'amino-3 propanediol-1,2.

3. Composition selon l'une quelconque des revendications 1 ou 2, **caractérisée par le fait que** le radical R₁ désigne un radical alkyle saturé ou insaturé, linéaire ou ramifié comportant de 8 à 18 atomes de carbone.

4. Composition selon l'une quelconque des revendications 1 à 3, **caractérisée par le fait que** le radical R₁ est un radical dérivé du coprah.

5. Composition selon l'une quelconque des revendications 1 à 4, **caractérisée par le fait que** les silicones se présentent sous forme d'huiles, de cires, de résines ou de gommes.

6. Composition selon l'une quelconque des revendications 1 à 5, **caractérisée par le fait que** les silicones sont choisies parmi :
- les polydiméthylsiloxanes à groupements terminaux triméthylsilyles
- les polydiméthylsiloxanes à groupements terminaux diméthylsilanols tels que les diméthiconols ;
- les polysiloxanes à groupements aminés tels que les amodiméthicones ou les triméthylsilylamodiméthicones ;

7. Composition selon l'une quelconque des revendications 1 à 6, **caractérisée en ce que** le tensioactif anionique de type hydroxy-2 alkyl éther carboxylique est présent dans les compositions à une concentration comprise entre 1 et 30 % en poids, de préférence entre 3 et 15% en poids par rapport au poids total de la composition.

8. Composition selon l'une quelconque des revendications 1 à 7, **caractérisée en ce que** ladite silicone est présente à une concentration comprise entre 0,001 % et 10 % en poids par rapport au poids total de la composition, de préférence entre 0,005 % et 5 % en poids et en particulier entre 0,01 % et 3 % en poids.

9. Composition selon l'une quelconque des revendications 1 à 8, **caractérisée par le fait qu'**elle comprend en outre au moins un agent tensioactif additionnel choisi parmi les tensioactifs anioniques, cationiques, non ioniques, amphotères et leurs mélanges.

10. Compositions selon la revendication 9, **caractérisées par le fait que** le ou les agents tensioactifs additionnels sont présents à une concentration comprise entre 0,5% et 40% en poids, de préférence entre 3% et 30% en poids, et encore plus préférentiellement entre 5% et 20% en poids, par rapport au poids total de la composition.

11. Composition selon l'une quelconque des revendications 1 à 10, **caractérisée par le fait qu'**elle comprend en outre au moins un additif choisi parmi les épaississants, les parfums, les agents nacrants, les conservateurs, les filtres solaires, les polymères anioniques ou non ioniques ou amphotères, les polymères cationiques , les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les huiles végétales, les huiles minérales, les huiles de synthèse, les agents antipelliculaires.

12. Composition selon la revendication 11, **caractérisée par le fait que** lesdits polymères cationiques sont choisis parmi :
- les homopolymères de sel de diallyldiméthylammonium et les copolymères de sel de diallyldiméthylammonium et d'acrylamide,
- les dérivés d'éther de cellulose quaternaires
- les polysaccharides cationiques
- les polymères qui sont constitués de motifs récurrents répondant à la formule : 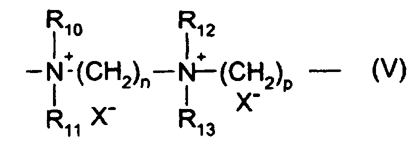
dans laquelle R₁₀, R₁₁, R₁₂ et R₁₃, identiques ou différents, désignent un radical alkyle ou hydroxyalkyle ayant de 1 à 4 atomes de carbone, n et p sont des nombres entiers variant de 2 à 20 et, X⁻ est un anion dérivé d'un acide minéral ou organique.

13. Composition selon l'une quelconque des revendications 1 à 12, **caractérisées par le fait qu'**elle se présente sous forme de shampooing, de composition lavantes pour la peau, d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

14. Utilisation d'une composition telle que définie dans l'une quelconque des revendications précédentes pour le lavage des matières kératiniques en particulier les cheveux.

15. Utilisation d'une composition telle que définie dans l'une quelconque des revendications précédentes pour le démêlage, le lissage des cheveux, pour apporter du volume, de la légèreté, de la douceur, de la souplesse et de la discipline aux cheveux..

16. Procédé de traitement des matières kératiniques, telles que les cheveux, **caractérisé en ce qu'**il consiste à appliquer sur lesdites matières une composition cosmétique selon l'une des revendications 1 à 13, puis à effectuer éventuellement un rinçage.

## Claims

1. Detergent cosmetic composition, **characterized in that** it comprises, in a cosmetically acceptable medium, at least one anionic surfactant of 2-hydroxyalkyl ether carboxylic acid type or its salts exhibiting the following structure: 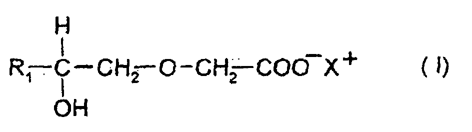 in which:
R₁ denotes a saturated or unsaturated, linear or branched alkyl radical comprising from 8 to 30 carbon atoms,
X denotes hydrogen or an inorganic or organic cation, such as those resulting from an alkali metal, NH₄⁺, ammoniums resulting from basic amino acids or ammoniums resulting from amino alcohols, and at least one silicone chosen from:
(i) volatile or non-volatile, linear, branched or cyclic and crosslinked or non-crosslinked polyalkylsiloxanes, polyarylsiloxanes or polyalkylarylsiloxanes,
(ii) polysiloxanes comprising, in their general structure, one or more organofunctional groups chosen from:
a) substituted or unsubstituted aminated groups
b) (per)fluorinated groups
c) thiol groups
d) carboxylate groups
e) hydroxylated groups
f) alkoxylated groups
g) acyloxyalkyl groups
h) amphoteric groups
i) bisulphite groups
j) hydroxyacylamino groups
k) carboxylic acid groups
1) sulphonic groups
m) sulphate or thiosulphate groups;
(iii) linear polysiloxane(A)-polyoxyalkylene(B) block copolymers of (A-B)ₙ type with n>3;
(iv) grafted silicone polymers with a non-silicone organic backbone, which polymers are composed of a main organic chain formed from organic monomers not comprising silicone, on which chain is grafted, inside the said chain and optionally at one at least of its ends, at least one polysiloxane macromonomer;
(v) grafted silicone polymers with a polysiloxane backbone which is grafted with non-silicone organic monomers, comprising a main polysiloxane chain on which is grafted, inside the said chain and optionally at one at least of its ends, at least one organic macromonomer not comprising silicone;
(vi) or their mixtures.

2. Composition according to Claim 1, **characterized in that** X denotes Na⁺, K⁺, NH₄⁺, ammoniums resulting from lysine, arginine, sarcosine, ornithine or citrulline, or ammoniums resulting from monoethanolamine, diethanolamine, triethanolamine, glucamine, N-methylglucamine or 3-amino-1,2-propanediol.

3. Composition according to either one of Claims 1 and 2, **characterized in that** the R₁ radical denotes a saturated or unsaturated, linear or branched alkyl radical comprising from 8 to 18 carbon atoms.

4. Composition according to any one of Claims 1 to 3, **characterized in that** the R₁ radical is a radical derived from copra.

5. Composition according to any one of Claims 1 to 4, **characterized in that** the silicones are provided in the form of oils, waxes, resins or gums.

6. Composition according to any one of Claims 1 to 5, **characterized in that** the silicones are chosen from:
- polydimethylsiloxanes with end trimethylsilyl groups,
- polydimethylsiloxanes with end dimethylsilanol groups, such as dimethiconols,
- polysiloxanes with aminated groups, such as amodimethicones or trimethylsilylamodimethicones.

7. Composition according to any one of Claims 1 to 6, **characterized in that** the anionic surfactant of 2-hydroxyalkyl ether carboxylic type is present in the compositions at a concentration of between 1 and 30% by weight, preferably between 3 and 15% by weight, with respect to the total weight of the composition.

8. Composition according to any one of Claims 1 to 7, **characterized in that** the said silicone is present at a concentration of between 0.001% and 10% by weight with respect to the total weight of the composition, preferably between 0.005% and 5% by weight and in particular between 0.01% and 3% by weight.

9. Composition according to any one of Claims 1 to 8, **characterized in that** it furthermore comprises at least one additional surface-active agent chosen from anionic, cationic, non-ionic or amphoteric surfactants and their mixtures.

10. Composition according to Claim 9, **characterized in that** the additional surface-active agent or agents are present at a concentration of between 0.5% and 40% by weight, preferably between 3% and 30% by weight and more preferably still between 5% and 20% by weight with respect to the total weight of the composition.

11. Composition according to any one of Claims 1 to 10, **characterized in that** it additionally comprises at least one additive chosen from thickeners, fragrances, pearlescent agents, preservatives, sunscreens, anionic or non-ionic or amphoteric polymers, cationic polymers, proteins, protein hydrolysates, ceramides, pseudoceramides, fatty acids with linear or branched C₁₆-C₄₀ chains, such as 18-methyleicosanoic acid, hydroxy acids, vitamins, panthenol, vegetable oils, mineral oils, synthetic oils or antidandruff agents.

12. Composition according to Claim 11, **characterized in that** the said cationic polymers are chosen from:
- diallyldimethylammonium salt homopolymers and copolymers of diallyldimethylammonium salt and of acrylamide,
- quaternary derivatives of cellulose ether,
- cationic polysaccharides,
- polymers which are composed of repeat units corresponding to the formula: 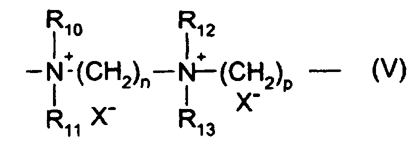
in which R₁₀, R₁₁, R₁₂ and R₁₃, which are identical or different, denote an alkyl or hydroxyalkyl radical having from 1 to 4 carbon atoms, n and p are integers varying from 2 to 20 and X⁻ is an anion derived from an inorganic or organic acid.

13. Composition according to any one of Claims 1 to 12, **characterized in that** it is provided in the form of a shampoo, of a composition for washing the skin, of a rinse-out or leave-in conditioner or of perming, hair-straightening, dyeing or bleaching compositions or in the form of rinse-out compositions, to be applied before or after a dyeing, bleaching, perming or hair straightening or between the two stages of a perming or hair straightening.

14. Use of a composition as defined in any one of the preceding claims in washing keratinous substances, in particular the hair.

15. Use of a composition as defined in any one of the preceding claims in disentangling or sleeking the hair or in contributing volume, lightness, softness, suppleness and manageability to the hair.

16. Process for the treatment of keratinous substances, such as the hair, **characterized in that** it consists in applying, to the said substances, a cosmetic composition according to one of Claims 1 to 13 and in then optionally rinsing.

## Patentansprüche

1. Reinigende kosmetische Zusammensetzung, **dadurch gekennzeichnet, dass** sie in einem kosmetisch akzeptablen Medium mindestens einen anionischen grenzflächenaktiven Stoff vom Typ der Carbonsäure-2-hydroxyalkylether oder ihrer Salze der folgenden Struktur: 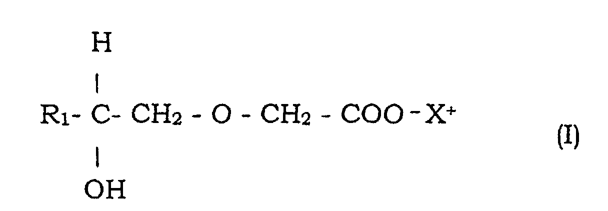 wobei die Gruppe R₁ eine geradkettige oder verzweigte, gesättigte oder ungesättigte Alkylgruppe mit 8 bis 30 Kohlenstoffatomen bedeutet, und
X Wasserstoff oder ein anorganisches oder organisches Kation bedeutet, wie z.B. die Kationen, die von einem Alkalimetall stammen, NH₄⁺, und Ammoniumionen, die von basischen Aminosäuren oder Aminoalkoholen abgeleitet sind,
und mindestens ein Silicon enthält, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
a) substituierten oder unsubstituierten aminierten Gruppen;
b) (per)fluorierten Gruppen;
c) Thiolgruppen;
d) Carboxylatgruppen;
e) hydroxylierten Gruppen;
f) alkoxylierten Gruppen;
g) Acyloxyalkylgruppen;
h) amphoteren Gruppen;
i) Bisulfitgruppen;
j) Hydroxyacylaminogruppen;
k) Carboxygruppen;
l) Sulfonsäuregruppen; und
m) Sulfat- oder Thiosulfatgruppen;
iii) linearen Polysiloxan(A)-Polyoxyalkylen(B)-Blockcopoylmeren vom Typ (A-B)ₙ mit n > 3;
iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
v) gepfropften Siliconpolymeren mit Polysiloxan-Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
vi) oder deren Gemischen.

2. Zusammensetzung nach Anspruch 1, **dadurch gekennzeichnet, dass** X Na⁺, K⁺, NH₄⁺ oder eine Ammoniumgruppe bedeutet, die von Lysin, Arginin, Sarcosin, Ornithin oder Citrullin abgeleitet ist oder die von Monoethanolamin, Diethanolamin, Triethanolamin, Glucamin, N-Methylglucamin oder 3-Amino-1,2-propandiol stammt.

3. Zusammensetzung nach einem der Ansprüche 1 oder 2, **dadurch gekennzeichnet, dass** die Gruppe R₁ eine geradkettige oder verzweigte, gesättigte oder ungesättigte Alkylgruppe mit 8 bis 18 Kohlenstoffatomen bedeutet.

4. Zusammensetzung nach einem der Ansprüche 1 bis 3, **dadurch gekennzeichnet, dass** die Gruppe R₁ eine von Kopra abgeleitete Gruppe ist.

5. Zusammensetzung nach einem der Ansprüche 1 bis 4, **dadurch gekennzeichnet, dass** die Silicone in Form von Ölen, Wachsen, Harzen oder Gummis vorliegen.

6. Zusammensetzung nach einem der Ansprüche 1 bis 5, **dadurch gekennzeichnet, dass** die Silicone ausgewählt sind unter:
- Polydimethylsiloxanen mit endständigen Trimethylsilylgruppen;
- Polydimethylsiloxanen mit endständigen Dimethylsilanolgruppen, wie Dimethiconen; und
- Polysiloxanen mit aminierten Gruppen, wie Amodimethiconen oder Trimethylsilylamodimethiconen.

7. Zusammensetzung nach einem der Ansprüche 1 bis 6, **dadurch gekennzeichnet, dass** der anionische grenzflächenaktive Stoff vom Typ der Carbonsäure-2-hydroxyalkylether in den Zusammensetzungen in einer Konzentration von 1 bis 30 Gew.-% und vorzugsweise 3 bis 15 Gew.-%, bezogen auf das Gesamtgewicht der Zusammensetzung, enthalten ist.

8. Zusammensetzung nach einem der Ansprüche 1 bis 7, **dadurch gekennzeichnet, dass** das Silicon in einer Konzentration von 0,001 bis 10 Gew.-%, vorzugsweise 0,005 bis 5 Gew.-% und insbesondere 0,01 bis 3 Gew.-%, bezogen auf das Gesamtgewicht der Zusammensetzung, enthalten ist.

9. Zusammensetzung nach einem der Ansprüche 1 bis 8, **dadurch gekennzeichnet, dass** sie ferner mindestens einen zusätzlichen grenzflächenaktiven Stoff enthält, der unter den anionischen, kationischen, nichtionischen oder amphoteren grenzflächenaktiven Stoffen und deren Gemischen ausgewählt ist.

10. Zusammensetzung nach Anspruch 9, **dadurch gekennzeichnet, dass** der oder die zusätzliche(n) grenzflächenaktive(n) Stoff(e) in einer Konzentration von 0,5 bis 40 Gew.-%, vorzugsweise 3 bis 30 Gew.-% und noch bevorzugter 5 bis 20 Gew.-%, bezogen auf das Gesamtgewicht der Zusammensetzung, vorliegen.

11. Zusammensetzung nach einem der Ansprüche 1 bis 10, **dadurch gekennzeichnet, dass** sie ferner mindestens einen Zusatzstoff enthält, der unter den Verdickungsmitteln, Parfums, Perlglanzpigmenten, Konservierungsmitteln, Sonnenschutzfiltern, anionischen, nichtionischen oder amphoteren Polymeren, kationischen Polymeren, Proteinen, Proteinhydrolysaten, Ceramiden, Pseudoceramiden, Fettsäuren mit geraden oder verzweigten C₁₆-₄₀-Ketten, beispielsweise 18-Methyleicosansäure, Hydroxysäuren, Vitaminen, Panthenol, pflanzlichen Ölen, Mineralölen, synthetischen Ölen und Antischuppenmitteln ausgewählt ist.

12. Zusammensetzung nach Anspruch 11, **dadurch gekennzeichnet, dass** die kationischen Polymere ausgewählt sind unter:
- Homopolymeren von Diallyldimethylammoniumsalzen und Copolymeren von Diallyldimethylammoniumsalzen und Acrylamid;
- quartären Celluloseetherderivaten;
- kationischen Polysacchariden;
- Polymeren die aus wiederkehrenden Einheiten der folgenden Formel bestehen: 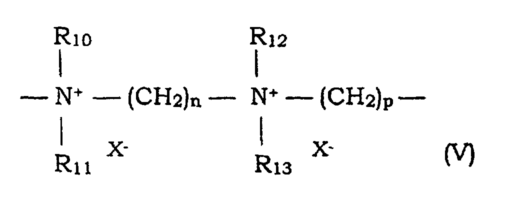
wobei die Gruppen R₁₀, R₁₁, R₁₂ und R₁₃, die identisch oder voneinander verschieden sind, eine Alkyl- oder Hydroxyalkylgruppe mit 1 bis 4 Kohlenstoffatomen bedeuten, n und p ganze Zahlen im Bereich von 2 bis 20 sind und X- ein von einer anorganischen oder organischen Säure abgeleitetes Anion ist.

13. Zusammensetzung nach einem der Ansprüche 1 bis 12, **dadurch gekennzeichnet, dass** es sich um ein Haarwaschmittel, eine Zusammensetzung für die Reinigung der Haut, ein Haarpflegemittel, das ausgespült wird oder im Haar verbleibt, eine Zusammensetzung für permanente Verformungen, Entkräuselungen, Färbungen oder Entfärbungen oder eine Zusammensetzung, die ausgespült wird und vor oder nach einer Färbung, Entfärbung, dauerhaften Verformung oder Entkräuselung oder zwischen den beiden Schritten einer dauerhaften Verformung oder Entkräuselung aufgetragen wird, handelt.

14. Verwendung einer Zusammensetzung nach einem der vorhergehenden Ansprüche zum Waschen von Keratinsubstanzen, insbesondere zum Waschen der Haare.

15. Verwendung einer Zusammensetzung nach einem der vorhergehenden Ansprüche zum Kämmen und Glätten der Haare und um den Haaren Volumen, Leichtigkeit, Weichheit, Geschmeidigkeit und Spannkraft zu geben.

16. Verfahren zur Behandlung von Keratinsubstanzen, wie dem Haar, **dadurch gekennzeichnet, dass** auf die Keratinsubstanzen eine kosmetische Zusammensetzung nach einem der Ansprüche 1 bis 13 aufgetragen und gegebenenfalls anschließend mit Wasser gespült wird.
